# EUROPEAN PATENT APPLICATION

(11) **EP 0 904 731 A2**
(43) Date of publication of application: **31.03.1999**
(21) Application number: 98307812.2
(22) Date of filing: 25.09.1998
(51) Int. Cl.: A61B 5/14

(54) **30 Gauge Lancet**

(30) Priority: 25.09.1997 US 937385
(71) Applicant: Becton, Dickinson and Company, Franklin Lakes, New Jersey 07417-1880 (US)
(72) Inventor: DiBiasi, Michael A., West Milford, NJ (US); West, Robert E., Morristown, NJ (US); Cramer, David J., Holdrege, NE (US); Stibick, Debra L., Albuquerque, New Mexico (US)
(74) Representative: Ruffles, Graham Keith

(57) **Abstract**

A lancet assembly includes an injection molded housing having a 30 Gauge metal stylet that is inserted and bonded therein. Since no molding forces are ever applied to the 30 Gauge stylet the resulting lancet assembly is the first having an entirely straight small diameter stylet. Therefore, the lancet assembly with this small straight 30 Gauge stylet causes less pain than any currently available larger gauge lancets. In addition, when the 30 Gauge stylet is lubricated after being mounted in the housing the lancet assembly not only causes less pain but has also been found to provide improved blood flow from the very small puncture wound.

## Description

### BACKGROUND OF THE INVENTION

### 1. Field of the Invention

The present invention relates to a lancet assembly and, more particularly, to a lancet assembly having an injection molded housing with a 30 Gauge metal stylet mounted therein.

### 2. Background Description

A lancet is a device commonly used in hospitals, doctors offices and homes to pierce a patient's flesh to draw capillary blood for diagnostic testing. Conventional lancets consist of a shank portion having at a distal end a blade or spike, which is sharp and adapted to pierce the patient's skin so to sever capillaries and provide blood for testing. Since the blade or spike is sharp, some lancets are provided with a removable shield for protectively covering the sharp edge or point of the lancet's blade or spike when not in use to protect the patient and users from inadvertent skin puncture.

There are a number of conventional lancet instruments available to aid a user in puncturing or penetrating a patient's skin to draw and sample a small outflow of blood. For example, U.S. Patent No. 4,577,630 (Nitzsche et al), which is assigned to Becton, Dickinson and Company, shows a disposable lancet assembly for use in a reusable breach loading target pressure activated lancet firing device. The lancet assembly includes a rectangular handle portion, a lancet point extending outwardly from a distal end of the handle portion, and a removable shield adapted to mate with the lancet point when the lancet assembly is not in use to protect users from accidental puncture.

U.S. Patent No. 4,517,978 (Levin et al.) shows another type of blood sampling instrument for drawing a blood sample from a patient's finger. The instrument described in Levin et al. includes a reusable tubular housing having a spring chamber for driving a needle held in a disposable needle holder into a patient's finger, when a trigger button on the side of the tubular housing is depressed. Prior to use, a disposable needle holder is slidably mounted in a socket within the tubular housing on a plunger member and the plunger member is slid to an armed position. The armed instrument is then placed against a patient's finger and activated using the trigger button. When activated the instrument drives the plunger towards the distal end of the instrument which causes the needle in the disposable needle holder on the plunger to pierce the patient's skin and create a drop of blood sufficient for testing.

More recently, U.S. Patent No. 5,569,286, which is also assigned to Becton, Dickinson and Company, is directed to anew lancet assembly having an injection molded housing with a 28 Gauge metal stylet mounted therein that is shielded by a cap prior to use. All of these earlier lancets continued to be found to cause users too much pain during the lancing procedure. Therefore, there is still the need to provide the user with a lancet that causes less pain, while still providing sufficient blood flow from the puncture wound after the lancing operation.

Until the present invention, those skilled in the art have found that reducing the diameter of the stylet has failed to solve the problem since the smaller puncture wound does not consistently provide sufficient blood flow and, more importantly, it has not been possible to manufacture an acceptable and defect-free lancet with such a small diameter stylet. In fact, injection molding a lancet around a smaller diameter or gauge stylet has been found to be unmanufactureable. For example, when insert molding a lancet having these smaller gauge stylets it has been found that the smaller gauge stylets warp or bend during the curing of the molded lancet. It appears that the forces applied to the stylet as the plastic cures causes the very thin metal stylet to bend, which results in a stylet extending from the lancet housing that is not straight. Of course, it is appreciated that such a bent stylet is entirely unacceptable because of the additional pain that such a stylet would cause to the user during the lancing operation as well as the problem it would cause when trying to insert the stylet into the blood sampling instrument.

### SUMMARY OF THE INVENTION

The present invention overcomes the problems identified in the background material by providing a lancet assembly for use in a blood sampling instrument having a housing with a 30 Gauge metal stylet.

The lancet assembly of the present invention includes an injected molded housing having a passageway for receiving a 30 Gauge stylet. After the housing has been manufactured, the 30 Gauge stylet is inserted and bonded into the passageway. Since no molding forces are ever applied to the 30 Gauge stylet the resulting lancet assembly is the first having an entirely straight small diameter stylet. Therefore, since the lancet assembly according to the present invention has such a small straight 30 Gauge stylet it has been found to cause less pain than any currently available larger gauge lancets. In addition, when the 30 Gauge stylet is lubricated after being mounted in the housing the lancet assembly of the present invention not only causes less pain but has also been found to provide improved blood flow from the very small puncture wound.

These and other aspects, features and advantages of the present invention will become apparent from the following detailed description taken in conjunction with the accompanying drawings.

### DESCRIPTION OF THE DRAWINGS

Fig. 1 is a perspective view of a shielded lancet assembly according to the present invention;
Fig. 2 is a perspective view of an unshielded lancet assembly according to the present invention;
Fig 3 is a top plan view of the cap shown in Fig. 2;
Fig. 4 is a bottom plan view of the cap shown in Figs. 2 and 3;
Fig 5 is a top plan view of the lancet assembly shown in Fig. 2;
Fig. 6 is a bottom plan view of the lancet assembly shown in Figs. 2 and 5; and
Fig. 7 is a cross-sectional view of the lancet assembly shown in Fig. 2, along line 7-7.

### DETAILED DESCRIPTION

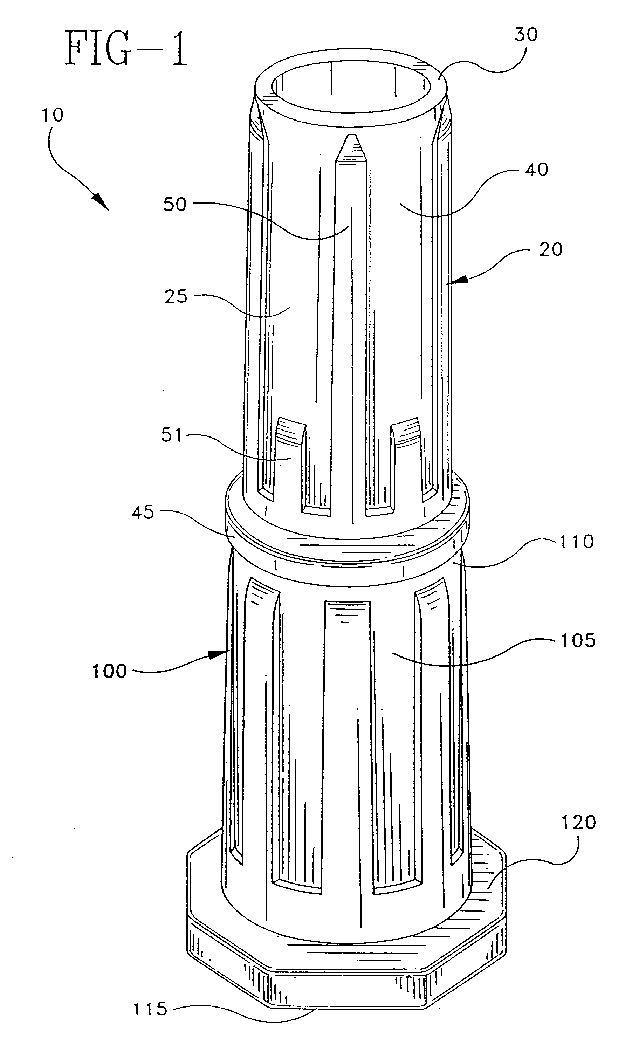

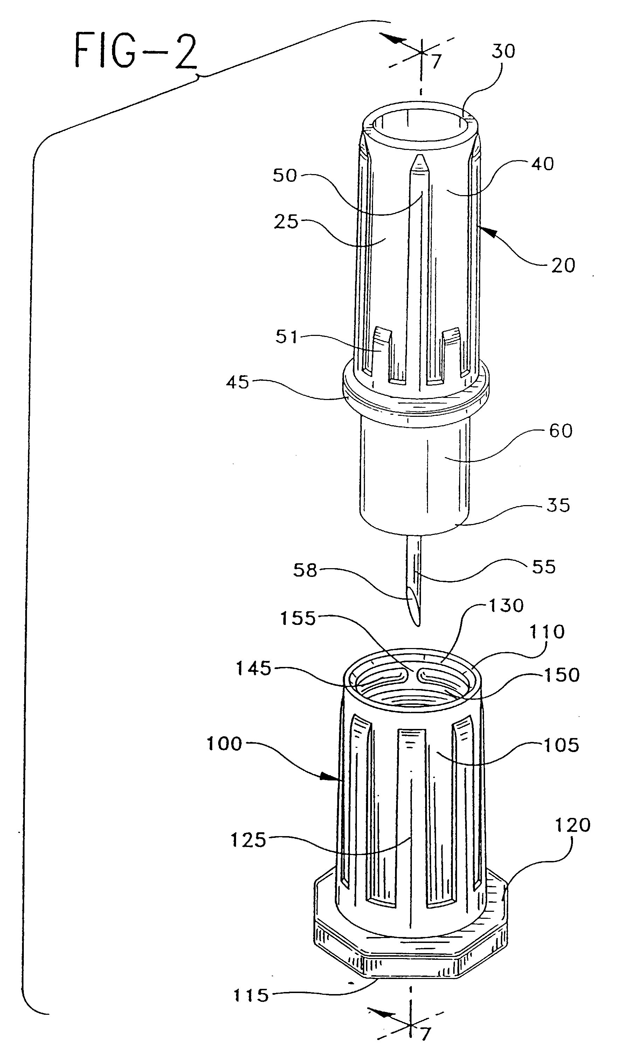

Fig. 1 is a perspective view of a shielded lancet assembly 10 according to the present invention for use in a blood sampling instrument similar to the one shown and described in U.S. Patent No. 4,517,978 (Levin et al.), included herein by reference. Shielded lancet assembly 10 includes a lancet assembly 20 with a removable cap or shield 100 attached thereto to shield and protect a user from a 30 Gauge stylet 55, shown in Fig. 2, in lancet assembly 20.

Lancet assembly 20 includes a cylindrical housing 25 having a proximal top end 30 and a distal bottom end 35, shown in Fig. 2, connected by an outer wall 40. An annular flange 45 extends from outer wall 40 and surrounds housing 25. A plurality of longitudinal ribs or splines 50 and 51 on outer wall 40 extend from flange 45 towards proximal end 30. Ribs 50 are longer than ribs 51 and alternate around outer wall 40 to provide a novel textured surface to improve handling, maneuverability and fit when lancet assembly 20 is mounted in a socket on the blood sampling instrument.

Removable cap 100 includes a cylindrical housing 105 having an open top end 110 and a closed bottom end 115. As shown in Fig. 2, distal bottom end 35 of lancet assembly 20 is received in open top end 110 of cap 100 to cover and shield 30 Gauge stylet 55 mounted in distal end 35 of lancet assembly 20. Closed bottom end 115 on cap 100 includes an octagonal shaped flange 120 extending radially therefrom that improves the removability of cap 100 from lancet assembly 20 and handling of cap 100. Removable cap 100 also includes a plurality of longitudinal ribs or splines 125 on housing 105 that extend from octagonal shaped flange 120 toward open end 110. Ribs 125 provide a sufficient textured surface to improve handling and maneuverability of cap 100, when it is being removed from lancet assembly 20.

When lancet assembly 20 is used in the conventional Levin et al. blood sampling instrument, a shielded lancet assembly 10 is inserted in the socket in the distal end of the Levin et al. instrument. Cap 100 is then removed from lancet assembly 20 using octagonal shaped flange 120 to unshield 30 Gauge stylet 55.

Fig 2 is an exploded perspective view of an unshielded lancet assembly according to the present invention. As shown in Fig. 2, 30 Gauge stylet 55 is bonded in lancet assembly 20 such that a sharpened point 58 on 30 Gauge stylet 55 extends from distal bottom end 35 of lancet assembly 20.

A user removes cap 100 from distal bottom end 35 of lancet assembly 20 by applying a longitudinal force to octagonal shaped flange 120 sufficient to slide a lower annular portion 60 at the base of 30 Gauge stylet 55 out of a chamber 130 in open top end 110 of cap 100. After the user has removed cap 100 from lancet assembly 20, the user positions the distal end of the Levin et al. instrument on the patient's finger and depresses the trigger button on the instrument. When the trigger button on the instrument is depressed, a spring chamber in the instrument drives 30 Gauge stylet 55 into the patient's finger.

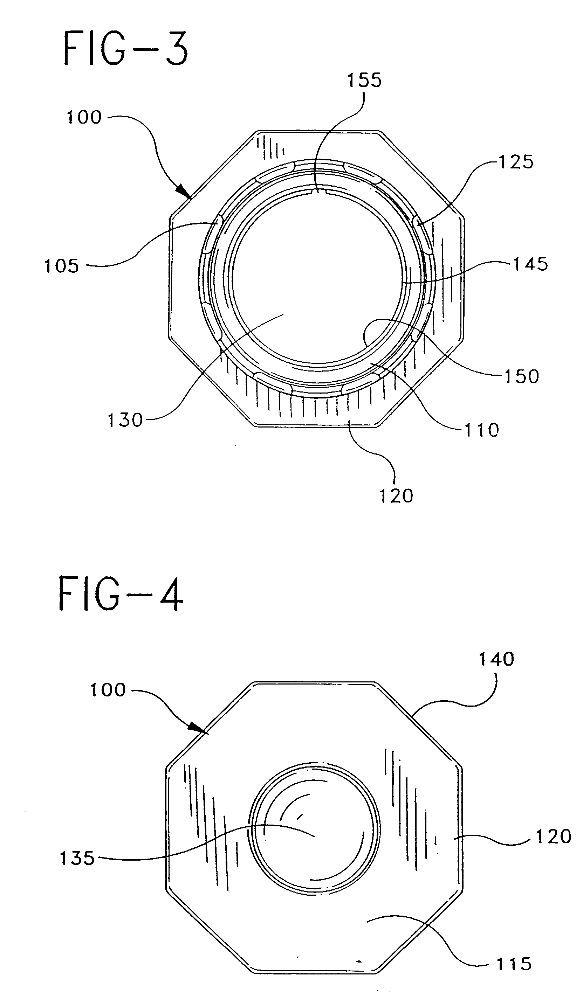

Fig. 3 is a top plan view of cap or shield 100, shown in Fig. 2. As shown, cap 100 includes chamber 130 at its center surrounded by cylindrical housing 105. Three labyrinth rings 145, each provided with a gap 155 separated from ring to ring by 120 degrees, are provided around an interior wall 150 in housing 105 to provide sterility and a labyrinth seal between rings 145 and housing 25 when attached to lancet assembly 20. The interference fit between rings 145 and housing 25 also determine the removal force necessary to remove cap 100 from lancet assembly 20. Fig. 3 also more clearly shows the positioning of ribs 125 around the circumference of housing 105 and the octagonal shape of flange 120. Fig. 4 is bottom a plan view of cap 100 and shows a concave circular center portion 135 in closed bottom end 115. It should be appreciated that the octagonal shape of flange 120 provides a plurality of surfaces 140 that permit a user to more easily grasp cap 100 when removing it from lancet assembly 20. Octagonal shaped flange 120 also provides a flat resting surface to cause cap 100 to stand upright with open top end 110 up, thereby allowing for reshielding of lancet assembly 20 after use without the user holding cap 100.

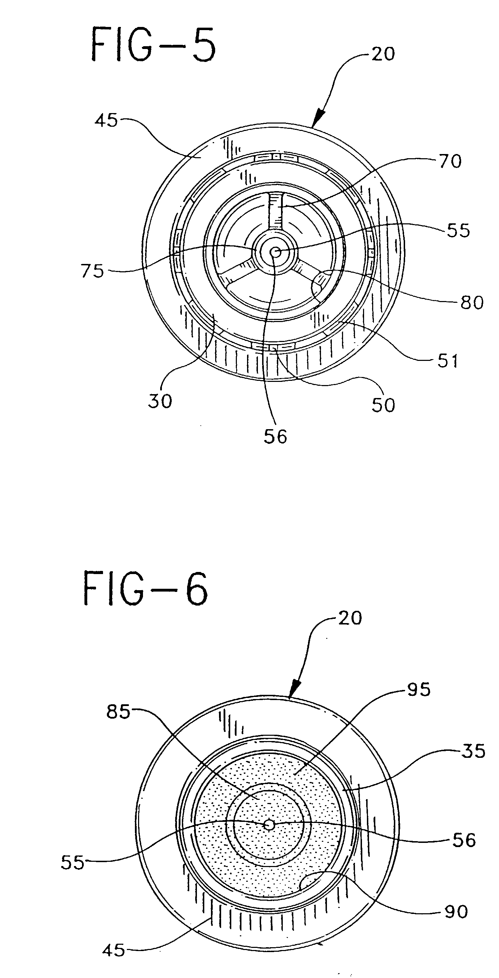

Fig. 5 is a top plan view of lancet assembly 20 that more clearly shows a proximal end of 30 Gauge stylet 55 mounted in passageway 56 and surrounded by a hub 75. Preferably, hub 75 is supported by a plurality of longitudinal supports 70 extending radially from hub 75. Each support 70 is separated from each other by 120 degrees and is attached to an inner wall 80 to securely hold and longitudinally position 30 Gauge stylet 55 in lancet assembly 20. 30 Gauge stylet 55 is bonded in passageway 56 such that it extends from distal end 35 by a predetermined distance. Fig. 6 is a bottom plan view of lancet assembly 20 that more clearly shows an adhesive 85 surrounding 30 Gauge stylet 55 in a reservoir 95 surrounded by an annular raised portion 90 for directing adhesive into passageway 56 when mounting 30 Gauge stylet 55. Preferably, adhesive 85 is a ultraviolet (UV) cured adhesive, but other types of adhesive may be used with the present invention. Since no molding forces are ever applied to 30 Gauge stylet 55 the resulting lancet assembly 20 is the first having an entirely straight small diameter stylet. In addition, since lancet assembly 20 has such a small and straight 30 Gauge stylet it causes less pain than any larger gauge lancet. It has also been found that if 30 Gauge stylet 55 is lubricated, which is performed after 30 Gauge stylet 55 has been bonded in passageway 56, lancet assembly 20 not only causes less pain but also provides improved blood flow from the very small puncture wound made by 30 Gauge stylet 55.

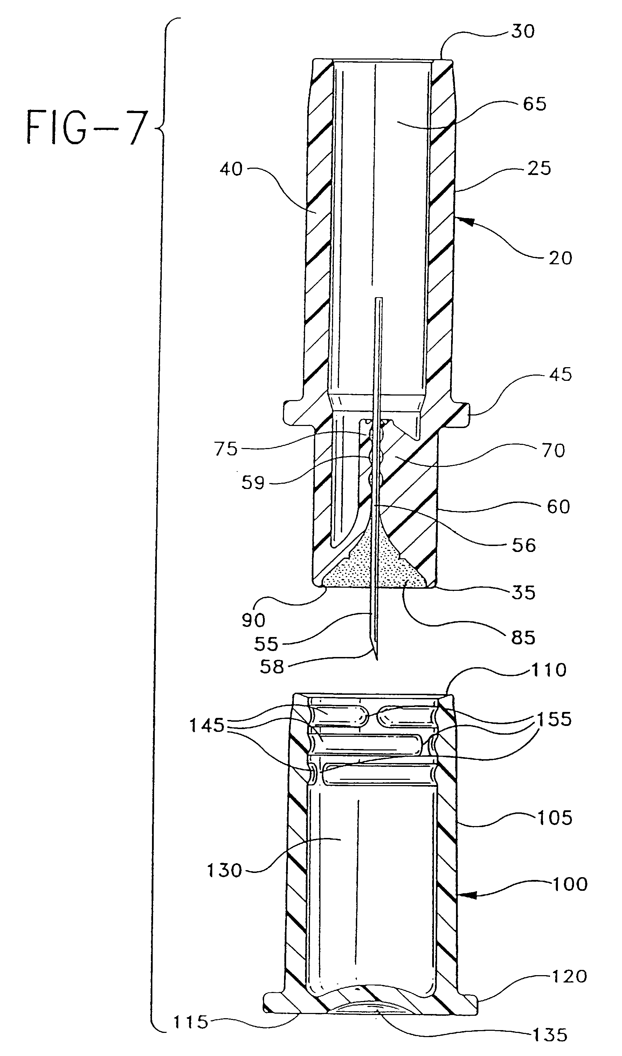

Fig. 7 is a cross-sectional view of lancet assembly 20 shown in Fig. 2, along line 7-7. As shown in Fig. 7, an upper chamber 65 extends from proximal top end 30 to a position level with flange 45 and just short of the entrance to passageway 56. Preferably, passageway 56 is shaped to receive 30 Gauge stylet 55 and includes a wavy surface 59 to provide an enhanced surface for bonding 30 Gauge stylet 55 in passageway 56. In addition, passageway 56 has an overall length sufficient to provide a straight stylet that is parallel to the longitudinal axis of housing 25. When cured, the adhesive forms a mechanical bond to the plastic passageway 56 and a chemical bond to the stainless steel 30 Gauge stylet 55 having holding power greater than the force required to pierce a patient's skin when drawing a blood sample. Fig. 7 also more clearly shows the shape of supports 70 within lower annular portion 60 and labyrinth rings 145 within cap 100, each ring having gap 155 therein. The gap in each adjacent ring is preferably separated by 120 degrees to provide a labyrinth seal around 30 Gauge stylet 55 when cap 100 is attached to lancet assembly 20.

As for manufacturing the lancet assemblies, housing 25 and cap 100 are injection molded from polypropylene and polyethylene, respectively. Applicants have found that using different materials in each part improves fit between the parts and makes it much easier to attach and remove cap 100 from housing 25. However, of course, these manufacturing techniques and materials are merely exemplary, various other manufacturing methods and materials could also be used.

In the foregoing discussion, it is to be understood that the above-described embodiment is simply illustrative of an improved lancet assembly, in accordance with the present invention. Other suitable variations and modifications could be made to these embodiments and still remain within the scope of the present invention.

## Claims

1. A lancet assembly comprising:
a housing having a proximal end and a distal end with a passageway contained therein; and
a 30 Gauge stylet mounted in said passageway and extending a predetermined distance from said distal end of said housing.

2. A lancet assembly according to Claim 1, wherein said 30 Gauge stylet is lubricated to improve blood flow from a puncture wound made by said 30 Gauge stylet.

3. A lancet assembly according to Claim 1, wherein said 30 Gauge stylet is bonded in said passageway.

4. A lancet assembly according to Claim 3, wherein said 30 Gauge stylet is lubricated to improve blood flow from a puncture wound made by said 30 Gauge stylet.

5. A lancet assembly according to Claim 1, wherein said housing is injection molded and said 30 Gauge stylet is inserted into and bonded in said passageway.

6. A lancet assembly according to Claim 5, wherein said 30 Gauge stylet is lubricated to improve blood flow from a puncture wound made by said 30 Gauge stylet.

7. A lancet assembly according to Claim 1, further comprising a cap for receiving said distal end of said housing to shield said 30 Gauge stylet when not in use

8. A lancet assembly according to Claim 7, wherein said cap comprises:
a cylindrical housing having an open top end and a closed bottom end, said housing being received in said open top end when said lancet assembly is shielded; and
a flange extending radially from said closed bottom end to aid a user in removing said cap from said housing and re-shielding said lancet assembly after use without holding said cap.
